**Europäisches Patentamt**

**European Patent Office**

**Office européen des brevets**

(19)

(11) Publication number : **0 303 681 B1**

# EUROPEAN PATENT SPECIFICATION

(12)

(45) Date of publication of patent specification :
**14.10.92 Bulletin 92/42**

(51) Int. Cl.⁵ : **A61K 37/02**

(21) Application number : **88902708.2**

(22) Date of filing : **23.02.88**

(86) International application number :
**PCT/US88/00576**

(87) International publication number :
**WO 88/06451 07.09.88 Gazette 88/20**

(54) **IMMUNOSUPPRESSION IN IMMUNOTOXIN BASED HUMAN THERAPY.**

(30) Priority : **24.02.87 US 18324**

(43) Date of publication of application :
**22.02.89 Bulletin 89/08**

(45) Publication of the grant of the patent :
**14.10.92 Bulletin 92/42**

(84) Designated Contracting States :
**AT BE CH DE FR GB IT LI LU NL SE**

(56) References cited :
**FR-A- 2 584 294**
**CHEMICAL ABSTRACTS, vol. 100, 1984, Columbus, OH (US) ; WEGNER, no. 155075k.**
**JOURNAL OF IMMUNOLOGY, vol. 134, no. 5, 05 May 1985 ; UCKUN, pp. 3504-3515.**
**IMMUNOLOGY, vol. 61, 1987, AU ; WARREN, pp. 167-172.**
**CANCER RESEARCH, vol. 47, 15 January 1985 ; WEIL-HILLMANN, pp. 579-585.**
**CANCER RESEARCH, vol. 45, January 1985 ; UCKUN, pp. 69-75.**

(73) Proprietor : **Xoma Corporation**
**2910 Seventh Street**
**Berkeley California 94710 (US)**

(72) Inventor : **MISCHAK, Ronald, P.**
**3095 Greer Road**
**Palo Alto, CA 94303 (US)**
Inventor : **SCANNON, Patrick, J.**
**Route 2, Box 2141**
**Davis, CA 95616 (US)**
Inventor : **SPITLER, Lynn, E.**
**71 Reed Ranch Road**
**Tiburon, CA 94920 (US)**
Inventor : **HARKONEN, W., Scott**
**3018 Sacramento Street**
**San Francisco, CA 94115 (US)**
Inventor : **MILLER, Langdon**
**379 - 15th Avenue**
**San Francisco, CA 94118 (US)**

(74) Representative : **Glawe, Delfs, Moll & Partner**
**Patentanwälte**
**Postfach 26 01 62 Liebherrstrasse 20**
**W-8000 München 26 (DE)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 303 681 B1

## Description

### Field Of The Invention

This invention relates to the use of immunosuppressive agents for producing a medicament for improving the effectiveness of immunotoxins in chemotherapy and other human treatment applications.

## BACKGROUND OF THE INVENTION

The development of hybridoma technology by Kohler and Milstein in 1975 was heralded as a major technological breakthrough for the fields of immunology and medicine. For the first time, researches were able to transform B-lymphocytes to create hybrid cells, with immortal potential, capable of secreting monoclonal antibodies, i.e., a single species of antibody reactive with a single type of epitope on a selected antigen. In practice, however, applying monoclonal antibody technology to improve chemotherapeutic and other therapies has been extremely difficult. In the decade since the discovery, very few therapeutic successes have been reported, despite extensive research efforts.

One reason for the lack of successful human therapeutic treatment regimes, at least in tumor therapy, is the fact that the mere binding of a monoclonal antibody to a target cell frequently does not induce cell death. To overcome this problem, there were considerable efforts devoted to coupling monoclonal antibodies to various cytotoxic agents capable of killing targeted cells. The monoclonal antibody would act as a "magic bullet", delivering the cytotoxic agent specifically to the desired cell. These immunoconjugates are known as "immunotoxins."

Uckun, F.M. et al., J. Immunol., 134:3504 (1985), report that treatment with a combination of immunotoxins and a cyclophosphamide (CY) derivative, mafosfamid, eliminates neoplastic T cells from an ex vivo human bone marrow culture. The combination of the two was apparently more effective for in vitro killing than either treatment alone.

Uckun, F.M. et al., Canc. Res., 45:69 (1985), describe that combining immunotoxin and CY (or CY derivatives) selectively eliminate leukemia cells in a bone marrow ex vivo culture, while 50 % of the pluripotent stem cells remain viable.

Weil-Hillman, et al., Canc. Res. 47:579 (1987), indicate that the combined use of an immunotoxin and mafosfamid can reduce human tumors in nude mice more than either agent alone.

While treatment studies with immunotoxins have been widespread and to some extent successful, the use of immunotoxins is often curtailed by a patient's immune response to both the monoclonal antibody and the toxin components of the immunotoxin. It is particularly thought to be a problem when the monoclonal antibody is of mouse or other non-human origin, although a patient's production of anti-idiotype antibodies could cause significant diminishment of the usefulness of immunotoxins even when made with human monoclonal antibodies.

An immune response against immunotoxin can cause premature removal of the immunotoxin from the patient's serum, significantly limiting the immunotoxin's effectiveness. Multiple treatment regimes are extremely susceptible to this problem, and increasing the immunotoxin dosage is generally undesirable, in part because the patient may suffer allergies and other harmful effects of immune reactions against the immunotoxin.

Thus, there is significant need for new methods of immunotoxin treatment, wherein the host immune response is abrogated or at least diminished to a level such that extended immunotoxin treatments are feasible. These methods of treatment should not include agents that are incompatible with immunotoxins or that will increase the amount of immunotoxin required to be administered to the patient. Ideally, the treatment methods will not substantially increase the toxicity of the overall therapy. The present invention fulfills these needs.

## SUMMARY OF THE INVENTION

The use of present invention provides novel methods for enhancing the effectiveness of imunotoxin based therapy in human patients, which methods comprise of an immunosuppressive dose of cyclophosphamide in conjunction with the immunotoxin. Alternatively, immunosuppressive doses of cyclosporin, 6-mercaptopurine and azathioprine may be used. These novel treatments have particular applicability in multiple dose treatment regimes using immunotoxin compositions, such as in chemotherapy when utilizing multiple injections of ricin A-chain conjugated to monoclonal antibodies specifically reactive with melanoma or other tumor associated antigens.

The immunosuppressive agent(s) can be administered concomitantly with the monoclonal antibody composition to obtain immunosuppression of at least about 50% to 85%. The dose will vary with each particular application, and typically the agent will be administered orally or as an intravenous injection. The doses will range widely, depending on the agent used and the administration interval, which can range from a single injection to multiple injections over a few days or greater than two weeks. In accordance with the present invention, various administration intervals and dosages which produce a substantially decreased immune response to the immunotoxin compositions are contemplated to permit multiple treatment regimes with the immunotoxin. If desired, cocktails containing additional compositions capable of adequately reducing the undesired immune response may be included.

## DESCRIPTION OF THE PREFERRED EMBODIMENTS

Novel methods are provided for improving immunotoxin based therapy in human patients by inhibiting the patient's immune response through the co-administration of at least one immunosuppressive dose of cyclophosphamide, cyclosporin, 6-mercaptopurine, or azathioprine with the immunotoxin. These agents, which can be utilized with prednisone, diminish the patient's immune response in a dose responsive manner and prolong the efficacy of the immunotoxin, enhancing the patient's prognosis for recovery. Moreover, in multiple immunotoxin treatment regimes, the total immunotoxin dose may be reduced.

Cyclophosphamide is a synthetic cytotoxic agent originally designed to improve the selectivity of alkylating compounds and is chemically related to the nitrogen mustards. It has a molecular formula of $C_7H_{15}Cl_2N_2O_2P.H_2O$, its molecular weight is 278.1 and its full chemical name is: 2-bis[(2chloroethyl)amino]- tetrahydro-2H-1,3,2-oxazaphosphorine 2-oxide monohydrate.Cyclophosphamide is available commercially and is sold, for example, under the registered trademarks CYTOXAN, by Bristol Meyers Corporation in Syracuse, New York, or NEOSAR, by Asta-Werke A.G., Bielefeld, Germany.

To be effective, cyclophosphamide is thought to require metabolic activation, and the drug and its metabolites are broadly distributed throughout the body after intravenous administration. Cyclophosphamide has a serum half life of about 4 hours, however, the drug and its metabolites are generally detectable in plasma up to 72 hours.

Cyclophosphamide is soluble in water, saline or ethanol and is suitable for parenteral or oral administration. Cyclophosphamide solutions may be given intravenously, intramuscularly, intraperitoneally, or intrapleurally. If desired, the solutions may be infused intravenously with dextrose and/or sodium chloride. [See, generally, Cancer Principles and Practice of Oncology, eds. DeVita et al, 2nd Ed. J. B. Lippincott Company, Philadelphia (1985).]

Appropriate dosages of cyclophosphamide (in deed, all of the immunosuppressive agents of the present invention) to achieve the desired immunosuppressive effect will vary to some extent depending on the individual patient's condition, on the particular immunotoxin composition administered, and on the patient's past exposure to the immunotoxin or related compositions. The suppression of the immune response is adequate to enable a second course of immunotoxin to be administered without neutralization or accelerated elimination by the patient's antibodies.

Treatment with cyclophosphamide and the other immunosuppressive agents is potentially hazardous to the patient, so continuous monitoring for harmful side effects should be maintained. In particular, a marked leukopenia is usually associated with significant doses. [See generally, Physicians Desk Reference, 42nd Edition (1988).]

Also, patients receiving certain immunotoxins have shown a reversible decrease in voltage on EKG not associated with either cardiac abnormalities and other constitutional symptoms that generally disappear within two weeks after treatment. Typically, there is no overlap between the toxicity of moderate doses of cyclophosphamide and the immunotoxin.

Another suitable agent, cyclosporine A, is a cyclic polypeptide ($C_{62}H_{111}N_{11}O_{12}$) immunosuppressant comprising 11 amino acids. It is produced as a metabolite by the fungus species Tolypocladium inflatum Gams.

Cyclosporine A is a potent immunosuppressive agent by an unknown mechanism of action, and is widely used in animals to prolong survival of allogeneic organ transplants. When administered in high doses, however, hepatoxicity and nephrotoxicity can result.

Cyclosporine A is soluble in ethanol and slightly soluble in water. It is typically administered orally or intravenously, and can be purchased under the trademark SANDIMMUNE by Sandoz, Ltd, Basle, Switzerland.

Other immunosuppressive agents include 6-mercaptopurine (MP) and its imidazolyl derivative azathioprine. The former is a well known antineoplastic and the latter an antimetabolite more commonly used in immunosuppression. MP is insoluble in water, but soluble in hot ethanol and alkaline solutions. Azathioprine is slightly soluble in water and ethanol. Both are available commercially, MP under the trademark PURINETHOL and azathioprine under the trademark IMURAN, from Burroughs Wellcome Corporation, Research Triangle Park, North Carolina, U.S.A.

Immunotoxins are characterized by two components. One component is the cytotoxic agent which is fatal to a cell when absorbed. The second component, known as the "delivery vehicle," provides a means for delivering the toxic agent to a particular cell type, such as cells comprising a carcinoma. The two components are commonly chemically bonded together by any of a variety of well-known chemical procedures. For example, when the cytoxic agent is a protein and the second component is an intact immunoglobulin, such as a monoclonal antibody, the linkage may be by way of hetero-bifunctional cross-linkers, e.g., SPDP, carbodiimide or gluteraldehyde. Production of various immunotoxins is well-known within the art, and can be found, for example, in "Monoclonal Antibody-Toxin Conjugates: Aiming the Magic Bullet", Thorpe et al, Monoclonal Antibodies in Clinical Medicine, Academic Press, pp 168-190 (1982).

A variety of cytotoxic agents are suitable for use in immunotoxins. Cytotoxic agents can include radionuclides, such as Iodine-131, Yttrium-90, Rhenium-188, and Bismuth-212; a number of chemotherapeutic drugs, such as vindesine, methotrexate, adriamycin, and cisplatinum; and cytotoxic proteins such as ribosomal inhibiting protein, pokeweed antiviral protein, abrin and ricin (or their A-chains, diphtheria toxin A-chains or pseudomonas exotoxin A) [See generally, "Chimeric Toxins", Olsnes and Phil, Pharmac. Ther., 25:355-381 (1982), and "Monoclonal Antibodies for Cancer Detection and Therapy," eds. Baldwin and Byers, pp. 159-179, 221-266, Academic Press (1985).]

The delivery vehicle component of the immunotoxin can be obtained from a number of sources. Intact immunoglobulins or their fragments, such as Fv, Fab or F(ab$_2$), may be used. Preferably, the immunoglobulins are monoclonal antibodies of the IgM or IgG isotype, of mouse, human or other mammalian origin.

A preferred source of monoclonal antibodies is immortalized murine or human cell lines thath may be cloned and screened in accordance with conventional techniques. However, recent technical advances have provided additional forms of immunoglobulins and methods of making them. For example, the utilization of recombinant DNA technology has produced functional, assembled immunoglobulins or hybrid immunoglobulins (e.g., the constant region from human monoclonal antibodies combined with mouse variable regions), suitable for use in immunotoxins. (See, e.g., EPA 84302368.0.)

Typically, the antibodies are capable of binding to epitopes of markers on selected cell types, such as neoplastic cells. The marker is generally a unique surface protein and a variety of markers, such as other proteins, glycoproteins, lipoproteins and polysaccharides, which are produced by the cells to be treated, can be utilized in accordance with the present invention. The general immunization fusion, screening, and expansion methods of monoclonal antibody technology are well-known to those skilled in the art and do not form part of the present invention.

A preferred method of measuring the immune response to an immunotoxin is based on the common ELISA assay. Briefly, microtiter plates are coated with the immunotoxin components, i.e., the immunoglobulin and the cytotoxic agent. After standard blocking and washing procedures, appropriate dilutions of patient's serum are added to the plate and any antibodies in the serum binding to the antigens are detected using an alkaline phosphatase conjugated goat anti-human antibody with heavy chain specificity for IgM or IgG. The antibody response is typically reported as a ratio. For each patient, the maximum measurable binding activity following therapy is determined by extrapolating the titration curve to the X axis. The response ratio is defined as a ratio of the titration end point value of the maximum response to the end point value of the patient's pre-treatment (baseline) serum. Other protocols may be substituted to assess the patient's immune response according to means well known by those skilled in the art.

By utilizing each patient's pre-treatment's serum as a baseline reference, positive responses to both the immunoglobulin and the cytotoxic agent components of the immunotoxin can be identified. Typically, the humoral aspect of the immune response is analyzed and a wide range of responses to each and/or both of the immunotoxin components is seen.

Based on control response studies and on data from various additional studies, an abrogation or prevention of the development of an immune response is indicated by a ratio less than about 2.0. An acceptable inhibition of a human immune response to either or both components of an immunotoxin would be preferably less than about 5.0 to 10 for both components, most preferably less than about 2.0 to 3.0.

To achieve a suitable reduction of a human immune response, a variety of dosage protocols may be followed, again, depending, e.g. upon the particular immunotoxin utilized and the condition of the patient. The amount of cyclophosphamide administered per injection may range from about 50 mg/m² up to about 1,500 mg/m² or more. Larger amounts per injection may be tolerated if the administration schedule calls for a single or a few injections. Lower amounts per injection may be administered over longer time periods of up to two weeks or more.

In one dose protocol, between 350 and 600 mg/m²/day, more preferably from about 400 to 550 mg/m²/day, and most preferably about 500 mg/m²/day, of cyclophosphamide is administered in five injections over a period of about 18 days. The initial three injections are preferably administered on alternate days on the first five days after treatment with immunotoxin, and the remaining two injections administered at four to six day intervals thereafter.

Alternatively, the cyclophosphamide may be administered in a single intravenous injection of between about 750 and 1,250 mg/m$^2$ preferably about 1,000/mg/m$^2$ between 4 and 24 hours or more after the initial immunotoxin injection. Yet another schedule entails administering about 14 daily injections, each of about 100 mg/m$^2$. Longer treatments with lower doses are preferred. Similarly, for cyclosporin A, the dose will typically range from about 3 to 15 mg/kg/day from 3 to 60 days. For 6-mercaptopurine, the usual dose will range from 1.5 to 7.5 mg/kg/day from 1 to 24 days. For azathioprine, the dose will range from 1 to 5 mg/kg/day for 3 to 60 days, typically in conjunction with prednisone at a dose range of 0.1 to 1.0 mg/kg/day over the same time period. If desired, the doses may be administered prior to the immunotoxin treatment, typically beginning about one week before such treatment.

An effective immunosuppresive dose would be an amount of immunosuppressive agent sufficient to limit the patient's immune response to a significantly reduced level of interference with the functional activity of the immunotoxin (e.g., to a level where a subsequent immunotoxin dose regimen retains substantial efficacy). This corresponds to the inhibiting the immune response in comparison to a normal response, ideally by about 85% to 95% or more, but inhibition levels of about 50% to 60% may be acceptable in some patients, and at least about 75% inhibition is preferred. Diminishment of various components of the immune response may be obtained, but reduction in antibody formation is a preferred result.

The period of co-administration of immunotoxin and immunosuppresive agent ideally should be coincided. In humans, the IgG response commonly can be detected about 7 to 8 days after exposure to the immunotoxin, and peaks at about 15 to 20 days or more thereafter. To minimize the IgG response, for example, the immunosuppressive agent is preferably administered primarily within the first few days after initial exposure to the immunotoxin. Subsequent doses may be administered to further abrogate this or other aspects of the immune response.

In this manner, prolonged or multiple treatment therapy regimes with the immunotoxin can be utilized to provide an increased level of therapeutic efficacy. Also, the total immunotoxin dose is minimized. Immunotoxin doses will vary widely according to the treatment.

Various combinations of immunotoxin and immunosuppressive agent administration schemes are acceptable for use with susceptible diseases, such as malignant tumors. The neoplasms suitable for treatment in accordance with the present invention include those neoplasms showing retarded growth or total remission when subjected to immunotoxin treatment. For example, such neoplasms include melanomas, gastrointestinal carcinomas, various leukemias, and T-cell and B-cell lymphomas. Aggressive grades of cancers are particularly suited for treatment with immunotoxins.

Immunotoxins also find use in other human therapies. By way of example, and not limitation, immunotoxins can be used to treat autoimmune diseases, graft rejection and graft versus host disease in allogeneic bone marrow transplantation, and graft rejection in other organ transplants.

Depending on the disease to be treated, cyclophosphamide, cyclosporine, 6-mercaptopurine and azathioprine may be used alone or with other immunosuppressive agents. These "cocktails" can be designed to universally and safely suppress immune responses in a wide variety of treatment regimes. Any of a variety of additional immunosuppressant agents known to the skilled artisan can be combined in the cocktail. For example, prednisone may be utilized with cyclophosphamide, azathioprine or cyclosporine at concentrations ranging from about 50 to 250 mg/m$^2$, preferably about 100 mg/m$^2$. Similarly, dexamethasone may be utilized with the cyclophosphamide at doses of about 5 to 30 mg, preferably about 15 mg. Typically, all of the immunosuppressive agents will be given coincidentally, such as both at day one, or both on five daily injections, but alternating administrations may be utilized. Actual methods for preparing and administering oral and parentenal compositions will be known or apparent to those skilled in the art and are described in detail, e.g., in Remington's Pharmaceutical Science, 16th Ed., Mack Publishing Co., Pennsylvania (1982).

The following examples are offered by way of illustration and not limitation.

EXPERIMENTAL

Example I

The immunotoxin utilized is XMMME-001-RTA as disclosed fully in U.S. Patent No. 4,590,071. The delivery portion of the immunotoxin is a monoclonal antibody secreted by a hybridoma designated XMMME-001, which was deposited with the American Type Culture Collection prior to the filing of the present application and designated Accession No. HB8759. This monoclonal antibody recognizes human melanoma associated antigen expressed on two glycoproteins with molecular weights of about 280 kd and about 440 kd. These antigens are expressed on a large majority of melanomas, a low percentage of squamous and basal cell carcinomas, but generally not in normal tissues of ectodermic, mesodermic, endodermic origin. The surface markers are heterogeneous in expression among lesions isolated from different patients and among sites within the tumor cell population.

The cytotoxic agent of this immunotoxin, labeled "RTA", consists of the ricin toxin A-chain, which has been separated from whole ricin in accordance with the teachings of U.S. Patent No. 4,590,071. The RTA is reduced and linked to the XMMME-001 antibody using succinimidyl 3-(2-pyridyldithio) propionate, which facilitates formation of disulphide bridges between the two components. The modified antibody is conjugated with the reduced ricin A-chain, purified, and then prepared for injection.

The administration of the XMMME-001-RTA is accomplished by slow intravenous infusion, generally completed within 30 minutes to one hour. It is generally not administered as an intravenous push or bolus.

In preliminary patient studies, clinical trials demonstrated that a single course of XMMME-001-RTA alone could be administered to patients safely and showed indications of efficacy in patients with metastatic melanoma. The use of cyclophosphamide alone for treatments of melanoma is unlikely to be effective (less than 13% response in a limited number of patients; see, Mastrangelo, et al in Cancer Principles and Practice of Oncology, eds. DeVita et al, J. P. Lippincott Co., Philadelphia pp 1,156 et. seq. (1985)).

Six patients having a documented history of metastatic malignant melanoma, Stage III disease, received 0.1 mg./kg/day of XMMME-001-RTA for five consecutive days in conjunction with cyclophosphamide. The first day of immunotoxin infusion is considered to be day one. Cyclophosphamide was administered by IV push over 2 to 10 minutes on days 2, 4 and 6, 12 or 13, and 16 or 17. On days when both cyclophosphamide and the immunotoxin were given, the cyclophosphamide dose was administered about 1 to 4 hours after the immunotoxin dose. There was no noticeable interaction between the immunotoxin and cyclophosphamide in their distribution, metabolism or excretion.

Three different doses of cyclophosphamide were administered. Three patients received 300 mg/m$^2$, two patients received 400 mg/m$^2$ (one of these received the cyclophosphamide for only the first three days), and one patient received 500 mg/m$^2$. Serum samples were collected prior to treatment and at weekly intervals for a minimum of four weeks. The samples were analyzed for antibody reactivity to the immunotoxin using a standard ELISA assay. Briefly, the assay entailed absorbing the XMMME-001 or ricin A-chain (RTA) to microtiter plates, which were then treated with glycine or bovine serum globulin for blocking. Appropriate dilutions of serum samples were incubated at 4° overnight and the amount of human immune globulin bound to the absorbed antigen was identified as IgM or IgG using alkaline phosatase conjugated heavy chain specific goat antihuman IgM or IgG. After an hour incubation with the antibody-enzyme conjugate, the substrate P-nitrophenylphosphate was added and incubated for one hour and optical densities (at 405 nm) were recorded.

The antibody response is reported as a response ratio. For each patient, the maximum measurable binding activity following therapy is determined by extrapolating the titration curve to the X axis. The response ratio is defined as the ratio of the titration end point value of the maximum response to the end point value of the patient's pre-treatment (baseline) serum.

By using each patient's pre-treatment serum as his/her baseline reference, we have been able to clearly identify positive responses to both the immunoglobulin and to the Ricin A Chain components of the immunotoxin (Table I). A wide range of response rates are demonstrated. The mean Ig ratio is 13.9 ± 15.9 SD. The mean A chain response ratio is 138.2 ± 208 SD. Thus, the response ratios were dispersed over a wide range and the ratios were generally considerably greater for an immune response to the ricin A-chain in comparison to the XMMME-001.

TABLE I

| | | RESPONSE TO XMMME-001 | | | RESPONSE TO RICIN A CHAIN | | |
|---|---|---|---|---|---|---|---|
| | | (Endpoint Dilution 1 x $10^{-3}$) | | | | | |
| Patient | IT Dose (mg/kg/day) | Baseline | Max | M/B | Baseline | Max | M/B |
| 1 | 0.50 | 3.2 | 30 | 9.4 | 0.1 | 10 | 100 |
| 2 | 0.50 | 2.0 | 50 | 25 | 0.1 | 24 | 240 |
| 3 | 0.50 | 50 | 100 | 2 | 0.1 | 5 | 50 |
| 4 | 0.5 | 6 | 200 | 33 | 1.2 | 200 | 166 |
| 5 | 0.50 | 3.2 | 5 | 1.6 | 1.6 | 20 | 12.5 |
| 6 | 0.50 | 2.4 | 10 | 4.2 | 1 | 300 | 300 |
| 7 | 0.75 | 2 | 4 | 2 | 1 | 1 | 1 |
| 8 | 0.75 | 1 | 1 | 1 | 1 | 1 | 1* |
| 9 | 0.50 | 1 | 40 | 40 | 0.2 | 50 | 250 |
| 10 | 0.20 | 1.5 | 13 | 8.6 | 3.2 | 10-100 | 3-33 |
| 11 | 0.75 | 1 | 1.6 | 1.6 | - | - | - |
| 12 | 0.50 | 1.2 | 10 | 8.3 | 0.4 | 80 | 200 |
| 13 | 0.20 | 0.4 | 1.6 | 4 | 0.3 | 200 | 666 |
| 14 | 0.75 | 2 | 16 | 8 | 0.2 | 160 | 800 |
| 15 | 1.0 | 0.4 | 9 | 22.5 | 0.15 | 2.5 | 16.7 |
| 16 | 1.0 | 2-10 | 100-64 | 50-6.4 | 2 | 10 | 5 |
| 17 | 0.50 | 0.4 | 1 | 2.5 | 0.1 | 6 | 60 |
| 18 | 0.50 | 3 | 100 | 33 | 3.2 | 150 | 46.8 |
| 19 | 0.20 | 0.25 | 0.45 | 1.8 | 0.2 | 9 | 45 |
| 20 | 0.20 | 0.4 | 3 | 7.5 | 0.4-0.1 | 8-7 | 20-70 |
| 21 | 0.05 | 2.5-1 | 125-10 | 50-10 | 0.5 | 40 | 80 |
| 22 | 0.01 | 0.7 | 1 | 1.4 | 0.7 | 8 | 12 |

* Samples to Day 3

The surprising results of cyclophosphamide addition inhibiting the IgG immune response are shown in Table II. The three patients receiving doses of 300 mg/m² showed a more regular base line reactivity than the patient in Table I. The mean ratio against the immunoglobulin was about 8.2, and against ricin A-chain the ratio was about 25. In addition, the response ratio to the ricin A-chain clustered in the range of about 20 to 30. In particular, cyclophosphamide substantially reduced the immune response in the patients receiving doses of 400 or 500 mg/m². In patient 5, there was a minimal IgG response to the monoclonal antibody, but this patient did not receive the full cyclophosphamide regime. Otherwise, the patients' response to ricin-A and XMMME-001 was either minimal or not detectable.

TABLE II

| Patient | Dose IT* | Dose CTX** | RESPONSE TO XMMME-001 (Endpoint Dilution 1 x 10⁻³) | | | RESPONSE TO RICIN A CHAIN | | |
|---|---|---|---|---|---|---|---|---|
| | | | Baseline | Max | M/B | Baseline | Max | M/B |
| A01 | 0.1 | 300 | 0.1 | 1.2 | 12 | 0.05 | 1.2 | 24 |
| A02 | 0.1 | 300 | 0.1 | 0.8 | 8 | 0.1 | 3.2 | 32 |
| A03 | 0.2 | 300 | 1.5 | 7 | 4.7 | 0.1 | 2 | 20 |
| A04 | 0.1 | 400 | 0.05 | 0.1 | 2 | 0.05 | 0.08 | 1.6 |
| A05 | 0.1 | 400 | 0.1 | 0.6 | 6 | 0.05 | 0.15 | 3 |
| A06 | 0.1 | 500 | 0.05 | 0.05 | 1 | 0.05 | 0.1 | 2 |

\* mg/kg/day

\*\* mg/m²

Example II

Utilizing the same procedures as in the prior example, the immunosuppressive capability of azathioprine, cyclosporine A and 6-mercaptopurine were analyzed in a separate clinical trial. The results are shown in Table III.

The dosage regimen for each immunosuppressive agent is indicated, each asterisk indicates an additional treatment with immunotoxins (treatments one and two were given four weeks apart and treatments two and three were given one to two weeks apart). The normal controls were tested to define the baseline variability in the assay system. In general, for IgG responses, serum dilutions of 1:100 to 1:1000 were required to give baseline OD values less than 0.1 and maximum end point titers ranged from 800 to 1600. In serum samples from 10 normal controls, response ratios remained tightly clustered around a value of one.

EP 0 303 681 B1

TABLE III

| Immunosuppressive Regimen | Patient | RESPONSE TO XMMME-001 | | RESPONSE TO RICIN A CHAIN | |
|---|---|---|---|---|---|
| | | Max | M/B | Max | M/B |
| Control | JC-01 | 6,000 | 60 | 2,400 | 6 |
| IT dose | BW-05 | 6,400 | 64 | 10,000 | 100 |
| 0.4 mg/kg x 1 | HS-30 | 1,200 | 12 | 800 | 8 |
| | RB-60 | 1,200 | 12 | 1,000 | 4 |
| | WG-201 | 6,400 | 9.1 | 800 | 8 |
| | AS-30 | 1,200 | 12 | 800 | 8 |
| Cyclophosphamide | TD-61 | 100 | 1 | 100 | 1 |
| $100 \ mg/m^2$/d x 14 | JR-62 | 400 | 2 | 3,200 | 2.9 |
| | HZ-63** | 100 | 1 | 100 | 1 |
| | Retreat | 1,600 | 16 | 100 | 1 |
| | Retreat | 2,000 | 3.3 | 100 | 1 |
| | SH-64 | 20,000 | 6.3 | 80,000 | 800 |
| Cyclophosphamide | JD-09 | 3,200 | 32 | 30,000 | 75 |
| $400 \ mg/m^2$ x 5 d | JK-10* | 6,400 | 6 | 5,000 | 16.6 |
| + | Retreat | 15,000 | 75 | --- | --- |
| Prednisone | HR-11 | 20,000 | 25 | 120,000 | 80 |
| 100 mg/d x 5 d | | | | | |

*Patient retreated
+Positive skin test before next treatment (treatment not given)

TABLE III (Continued)

| Immunosuppressive Regimen | Patient | Max | M/B | Max | M/B |
|---|---|---|---|---|---|
| Cyclophosphamide 1000 mg/m$^2$ x 1 Same day as IT | JO-100 | 1,600 | 8 | 6,000 | 6.7 |
| | BR-101 | 11,000 | 110 | 3,200 | 32 |
| | JE-120 | 10,000 | 100 | 3,200 | 32 |
| | LS-121* | 3,300 | 33 | 2,000 | 20 |
| | TR-105 | 25,000 | 25 | 15,000 | 150 |
| | LG-122 | 12,000 | 7.5 | 100 | 1 |
| | KN-124+ | 10,000 | 12.5 | 10,000 | 100 |
| | WC-123 | 30,000 | 18 | 8,000 | 40 |
| | SA-125 | 20,000 | 20 | 50,000 | 250 |
| Cyclophosphamide 1000 mg/m$^2$ single dose day after IT | RR-31 | 25,000 | 8.1 | 160,000 | 289 |
| | RY-32 | 20,000 | 8 | 40,000 | 400 |
| | LR-33 | 12,500 | 31.2 | 60,000 | 7.5 |
| Azathioprine 3 mg/mk/d x 56 d + Prednisone 0.24 mg/kg/d x 56 d | WR-06* | 1,000 | 1 | 800 | 8 |
| | Retreat | 1,500 | 1.8 | 3,200 | 32 |
| | MJ-07** | 800 | 8 | 100 | 1 |
| | Retreat | 11,000 | 27.5 | 3,000 | 30 |
| | Retreat | 50,000 | 50.5 | 50,000 | 500 |
| | EP-08* | 3,000 | 8.6 | 8,000 | 80 |
| | Retreat | 25,000 | 31.25 | 40,000 | 400 |

*Patient retreated
+Positive skin test before next treatment (treatment not given)

EP 0 303 681 B1

TABLE III (Continued)

| Immunosuppressive Regimen | Patient | Max | M/B | Max | M/B |
|---|---|---|---|---|---|
| Azathioprine 3 mg/kg/d x 56 d Predisone 0.24 mg/kg/d x 56 d Start 1 week before IT | SC-12 | 20,000 | 5 | 80,000 | 400 |
| Cyclosporin Daily continuously for 60 days 10-15 mg/kg/day | DM-140*** | 1,000 | 1 | 1,000 | 1 |
| | RS-141**+ | 10,000 | 1.6 | 800 | 1 |
| | PS-142** | 3,200 | 1 | 200 | 1 |
| | TW-143*** | 1,200 | 1.7 | 200 | 1 |
| | PS-144*** | 2,000 | 1.7 | 3,200 | 32 |
| 6 MP:2.5 mg/kg Daily for 21 days | WJ-202* | 5,000 | 5 | 8,000 | 1 |
| | Retreat | 6,400 | 1.6 | 6,400 | 2 |
| | HA-203* | 800 | 1 | 1,600 | 1 |
| | GH 207 | 10,000 | 6.2 | 1,600 | 8 |
| 6 MP:7.5 mg/kg Daily for 7 days | LK-204 | 10,000 | 6.2 | 10,000 | 12.5 |
| | ET-205 | 3,000 | 1.9 | 800 | 8 |
| | MS-206 | 3,200 | (32) | 15,000 | (150) |

*Patient retreated
+Positive skin test before next treatment (treatment not given)
( ): Estimate: no baseline sample

TABLE III (Continued)

| Immunosuppressive Regimen | Patient | Max | M/B | Max | M/B |
|---|---|---|---|---|---|
| Cyclophosphamide 500 mg/m² x 3 days + Prednisone 100 mg/day x 3 days | DK-180* | 20,000 | 10 | 1,200 | 1.5 |
| | Retreat | 50,000 | 25 | 6,400 | 8 |
| | DH-181 | 12,000 | 7 | 500 | 5 |

*Patient retreated
+Positive skin test before next treatment (treatment not given)

From the foregoing, it will be appreciated that the use of cyclophosphamide, cyclosporine A, azathioprine and 6-mercaptopurine in concurrent therapy with an immunotoxin substantially reduces the patient's immune response to the immunotoxin components. Thus, multiple immunotoxin treatments are feasible, which increases the effectiveness of immunotoxin therapy and improves the patient's prognosis from the treatment. Moreover, allergic reactions and other harmful aspects of an immune response generated against the immunotoxin are diminished, typically without substantially increasing the toxicity of the overall treatment program.

Although the present invention has been described in some detail by way of example for purposes of clarity and understanding, it will be apparent that certain changes and modifications may be practiced with in the scope of the appended claims.

## Claims

1. The use of at least one immunosuppressant selected from cyclophosphamide, cyclosporine A, azathioprine and 6-mercaptopurine for producing a medicament for increasing the effectiveness of immunotoxin based therapy in a human patient undergoing multiple treatment regimens with said immunotoxin.

2. The use according to claim 1 wherein said immunosuppressant is cyclophosphamide.

3. The use of cyclophosphamide for producing a medicament for inhibiting a human immune response against one or more injections of an immunotoxin over a predetermined treatment protocol, said medicament being prepared for co-administration of a single immuno-suppressive dose of cyclophosphamide.

4. The use according to anyone of the claims 1 to 3 for treatment of a tumor, wherein said immunotoxin comprises a cytotoxic agent bound to an antibody specifically reactive with a marker on said tumor.

5. The use according to anyone of the claims 1 to 4 for treating metastatic malignant melanoma in a human patient wherein said immunotoxin is XMMME-001-RTA.

6. The use according to anyone of the claims 1 to 5 for administration with a therapeutically effective dose of prednisone, dexamethasone and/or mixtures thereof.

7. The use according to anyone of the claims 1 to 6 for reducing an immune response of said patient against the immunotoxin by at least 50 percent from a normal immune response.

## Patentansprüche

1. Verwendung von mindestens einem immunosupprimierenden Mittel, ausgewählt aus Cyclophosphamid, Cyclosporin A, Azathioprin und 6-Mercaptopurin, zur Herstellung eines Arzneimittels zur Steigerung der Wirksamkeit einer immunotoxin-basierenden Therapie bei einem menschlichen Patienten, der mehrfachen Behandlungsvorschriften mit dem genannten Immunotoxin unterworfen wird.

2. Verwendung nach Anspruch 1, bei der das immunosupprimierende Mittel Cyclophosphamid ist.

3. Verwendung von Cyclophosphamid zur Herstellung eines Arzneimittels zur Inhibierung einer menschlichen Immunreaktion gegen eine Injektion oder mehrere eines Immunotoxins über ein vorbestimmtes Behandlungsprotokoll, wobei das Medikament zur Verabreichung zusammen mit einer einzelnen, immunosupprimierenden Dosis von Cyclophosphamid hergestellt worden ist.

4. Verwendung nach einem jeden der Ansprüche 1 bis 3 zur Behandlung eines Tumors, wobei das genannte Immunotoxin ein cytotoxisches Mittel umfaßt, das an einen Antikörper gebunden ist, der zu einer spezifischen Reaktion mit einem Marker auf dem Tumor befähigt ist.

5. Verwendung nach einem jeden der Ansprüche 1 bis 4 zur Behandlung von metastatischen, malignen Melanomen bei einem menschlichen Patienten, wobei das Immunotoxin XMMME-001-RTA ist.

6. Verwendung gemäß einem jeden der Ansprüche 1 bis 5 zur Verabreichung mit einer therapeutisch wirksamen Dosis von Prednison, Dexamethason und/oder Mischungen derselben.

7. Verwendung gemäß einem jeden der Ansprüche 1 bis 6 zur Verringerung einer Immunreaktion des Patienten gegen das Immunotoxin um mindestens 50 % von einer normalen Immunreaktion.

**Revendications**

1. L'utilisation d'au moins un immunosuppresseur choisi parmi le cyclophosphamide, la cyclosporine A, l'azathioprine et la 6-mercaptopurine pour la fabrication d'un médicament pour augmenter l'efficacité de la thérapie à base d'immunotoxine chez un patient recevant des traitements multiples au moyen de ladite immunotoxine.

2. L'utilisation selon la revendication 1, dans laquelle ledit immunosuppresseur est le cyclophosphamide.

3. L'utilisation du cyclophosphamide pour la fabrication d'un médicament pour inhiber une réponse immune humaine contre une ou plusieurs injections d'une immunotoxine suivant un protocole de traitement déterminé, ledit médicament étant préparé pour la co-administration d'une dose immunosuppressive unique de cyclophosphamide.

4. L'utilisation selon l'une quelconque des revendications 1 à 3 pour le traitement d'une tumeur, dans laquelle ladite immunotoxine comprend un agent cytotoxique lié à un anticorps réagissant spécifiquement avec un marqueur sur ladite tumeur.

5. L'utilisation selon l'une quelconque des revendications 1 à 4 pour le traitement de mélanomes métastatiques malins chez un patient humain, dans laquelle ladite immunotoxine est XMMME-001-RTA.

6. L'utilisation selon l'une quelconque des revendications 1 à 5 pour l'administration avec une dose thérapeutiquement efficace de prednisone, de dexaméthasone et/ou de leurs mélanges.

7. L'utilisation selon l'une quelconque des revendications 1 à 6 pour la réduction d'une réponse immune dudit patient contre l'immunotoxine, d'au moins 50 pourcent d'une réponse immune normale.